# EUROPEAN PATENT SPECIFICATION

(11) **EP 3 941 460 B1**
(45) Date of publication and mention of the grant of the patent: **15.10.2025**
(21) Application number: 20774479.8
(22) Date of filing: 09.03.2020
(51) Int. Cl.: A61K 31/567, A61K 31/4745, A61K 31/513, A61K 31/57, A61K 31/585, A61K 31/365, A61K 45/06, A61P 3/04, A61P 3/06, A61P 3/08, A61P 3/10

(54) **MIFEPRISTONE FOR USE IN IMPROVING INSULIN SENSITIVITY**
MIFEPRISTONE ZUR VERWENDUNG BEI DER VERBESSERUNG DER INSULINEMPFINDLICHKEIT
MIFEPRISTONE POUR UTILISATION DANS L'AMÉLIORATION DE LA SENSIBILITÉ À L'INSULINE

(30) Priority: 18.03.2019 US 201962919496 P
(43) Date of publication of application: 26.01.2022
(73) Proprietor: Nieman, Lynnette K., Bethesda, MD 20892 (US); Ulmann, André, 75005 Paris (FR); Newman, Arnold L., Cabin John, MD 20818 (US)
(72) Inventor: Nieman, Lynnette K., Bethesda, MD 20892 (US); Ulmann, André, 75005 Paris (FR); Newman, Arnold L., Cabin John, MD 20818 (US)
(74) Representative: Cabinet Becker et Associés
(86) International application number: PCT/US2020/000009
(87) International publication number: WO 2020/190351

(56) References cited:
- WO-A1-01/52833
- WO-A1-03/009853
- CN-A- 102 204 918
- US-A- 5 073 548
- US-A- 6 166 013
- US-A1- 2002 147 336
- US-A1- 2002 147 336
- US-A1- 2004 214 804
- US-A1- 2005 080 061
- US-A1- 2009 088 425
- US-A1- 2009 088 425
- US-A1- 2012 028 358
- DEBONO MIGUEL ET AL: "Mifepristone Reduces Insulin Resistance in Patient Volunteers with Adrenal Incidentalomas That Secrete Low Levels of Cortisol: A Pilot Study", vol. 8, no. 4, 1 January 2013 (2013-01-01), pages e60984, XP055981535, Retrieved from the Internet <URL:https://journals.plos.org/plosone/article/file?id=10.1371/journal.pone.0060984&type=printable> DOI: 10.1371/journal.pone.0060984
- ANONYMOUS: "History of Changes for Study: NCT03052400 Efficacy of Mifepristone in Males With Type 2 Diabetes Mellitus", CLINICALTRIALS.GOV ARCHIVE, 1 January 2017 (2017-01-01), pages 1 - 7, XP055981665, Retrieved from the Internet <URL:https://www.clinicaltrials.gov/ct2/history/NCT03052400?V_2=View#StudyPageTop> [retrieved on 20221115]
- CHU J W ET AL: "Successful long-term treatment of refractory Cushing's disease with high-dose mifepristone (RU 486)", JOURNAL OF CLINICAL ENDOCRINOLOGY AND METABOLISM, THE ENDOCRINE SOCIETY, US, vol. 86, no. 8, 1 August 2001 (2001-08-01), pages 3568 - 3573, XP002410091, ISSN: 0021-972X, DOI: 10.1210/JC.86.8.3568
- GUBBI SRIRAM ET AL: "Mifepristone Improves Adipose Tissue Insulin Sensitivity in Insulin Resistant Individuals", vol. 106, no. 5, 23 April 2021 (2021-04-23), US, pages 1501 - 1515, XP055981608, ISSN: 0021-972X, Retrieved from the Internet <URL:https://www.ncbi.nlm.nih.gov/pmc/articles/PMC8063260/> DOI: 10.1210/clinem/dgab046
- CLARK ROBIN: "Glucocorticoid Receptor Antagonists", CURRENT TOPICS IN MEDICINAL CHEMISTRY, vol. 8, no. 9, 1 June 2008 (2008-06-01), NL, pages 813 - 838, XP093138635, ISSN: 1568-0266, Retrieved from the Internet <URL:https://www.eurekaselect.com/article/27705> DOI: 10.2174/156802608784535011
- YOSUP REW ET AL: "Discovery of a Potent and Selective Steroidal Glucocorticoid Receptor Antagonist (ORIC-101)", JOURNAL OF MEDICINAL CHEMISTRY, vol. 61, no. 17, 9 August 2018 (2018-08-09), US, pages 7767 - 7784, XP055605922, ISSN: 0022-2623, DOI: 10.1021/acs.jmedchem.8b00743
- "miricorilantum", WHO DRUG INFORMATION. PROPOSED INN: LIST 119, vol. 32, no. 2, 2018, pages 283 - 398, XP055741138
- CLORE ET AL.: "When Is Cortisol a Mineralocorticoid?", KIDNEY INTERNATIONAL, vol. 42, no. 6, December 1992 (1992-12-01), pages 1297 - 1308, XP055741141, DOI: 10.1038/ki.1992.421

## Description

### BACKGROUND OF THE INVENTION

Insulin sensitivity is a measure of the degree to which cells respond to insulin. The converse, insulin insensitivity, (also termed insulin resistance), is a pathological condition wherein the cellular response to the presence of insulin in the bloodstream is diminished to the extent that homeostasis cannot be maintained and the normal metabolism of glucose is disrupted.

Normally, insulin causes cells to take up glucose, which is used as an energy source. Thus, insulin is a critical part of the process by which normal levels of blood glucose are maintained. If cells become less sensitive to insulin, that is, insulin resistant, they become less effective in taking up glucose and blood glucose levels rise beyond normal levels. This reduced activity of insulin may cause prediabetes, diabetes, metabolic syndrome, non-alcoholic fatty liver disease and other diseases of metabolism.

Insulin sensitivity and resistance may occur differentially in the various tissues that are involved in glucose and fat metabolism, including, but not limited to, the liver, skeletal muscles and adipose tissues. Each of these contributes to "total" or overall insulin insensitivity and resistance, and prolonged changes in hepatic and adipose insulin sensitivity lead to changes in total body insulin sensitivity.

Glucocorticoids include corticosteroid hormones produced in the adrenal cortex, as well as synthetic analogs of these hormones. In addition to other physiologic activities, glucocorticoids are involved in carbohydrate (e.g., glucose) fat and protein metabolism. Cortisol is the main glucocorticoid in humans.

Among other actions, glucocorticoids have been shown to stimulate gluconeogenesis, mobilize amino acids, and inhibit glucose uptake in muscle and fat. Chronically increased blood levels of the glucocorticoid, cortisol, occur in Cushing's Syndrome. The increased glucocorticoid action in this condition is implicated in abnormal glucose and fat metabolism leading to obesity, metabolic syndrome and diabetes.

Glucocorticoids exert their actions by binding to the glucocorticoid receptor, which is a ligand activated nuclear receptor. The ligand-bound-receptor translocates to the nucleus where it binds to specific sites on DNA, leading to RNA transcription and then protein synthesis. It has been shown that the effects resulting from glucocorticoid binding to the glucocorticoid receptor can be inhibited at least partially by the administration of anti-glucocorticoids, that is, synthetic molecules that bind to the glucocorticoid receptor and prevent it from acting on DNA to induce transcription.

Appropriate levels of cortisol are critical for human homeostasis and normal metabolism, which may explain the tight regulation of its production. Cortisol biosynthesis and secretion are regulated by adrenocorticotropic hormone (ACTH) produced in the pituitary gland, which in turn is stimulated by corticotropin-releasing hormone (CRH), produced and secreted by the hypothalamus. Cortisol and ACTH levels (and by inference CRH levels) are lowest after the onset of sleep, gradually rising before wakening and falling beginning a few hours thereafter. This circadian pattern appears to be critical to health. While the factors that activate the system are not fully understood, it is clear that increasing cortisol levels have a "negative feedback" effect to inhibit ACTH secretion, so that cortisol partially governs its own rate of secretion.

Currently, the number of people who are characterized as being overweight and/or obese is increasing dramatically. This represents a major healthcare challenge due also to the associated co-morbidities of hypertension, hyperlipidemia and glucose intolerance/diabetes. Hence, there is an unmet and critical need to reverse elements of metabolic syndrome, including hypertension, weight gain, hyperlipidemia and glucose intolerance/diabetes as pre-diabetes (pre-DM) and overt diabetes mellitus (DM) continue to exert substantial physical and financial burden on patients worldwide.

The estimated annual incidence of diabetes in the United States population is projected to increase from 8 cases per 1,000 in 2008 to 15 cases per 1,000 by 2050, and the prevalence is projected to rise from 14% in 2010 to 25-28% by 2050. Uncontrolled diabetes accounts for about 10% of deaths, in part from its comorbidities of cardiovascular disease and renal failure. In view of these current statistics and projections, provision of novel treatments to improve glucose tolerance and diabetes are urgently needed to prevent progression of pre-diabetes and to reduce the morbidity and mortality of diabetes.
International patent application WO2003/009853 discloses administering glucocorticoid receptor antagonists in patients who suffer from weight induced by anti-psychotic drugs.
Debono et al, PLOS ONE, 2013, 8(4) page e60984 reports that mifepristone reduces insulin resistance in patient volunteers with adrenal incidentalomas that secrete low levels of cortisol.
International patent application WO2001/52833 discloses a controlled release of a cortisol antagonist in treating metabolic syndrome or type 2 diabetes.
Clinical trial NCT03052400 announces administration of mifepristone, 600mg daily, in males with type 2 diabetes (clinicaltrials.gov; January 2017, pages 1-7).

### SUMMARY OF THE INVENTION

The invention is as defined in the appended claims.

It is herein described a glucocorticoid receptor antagonist that is mifepristone, for use in a method of ameliorating a disorder of insulin sensitivity by administration of mifepristone in an amount which does not activate the hypothalamic-pituitary-adrenal (HPA) axis, or increases daily urine cortisol secretion no more than about three times the upper limit of the normal range, or increases 24-hour mean serum cortisol or area under the curve (AUC) no more than twice normal.

The disorder may be e.g. diabetes, prediabetes, lipodystrophy, hypertriglyceridemia, and/or growth hormone excess states. It is also an object of the present invention to improve insulin sensitivity in individuals who are neither overweight nor obese.

It is further described a method for improving insulin sensitivity of a patient by blocking or otherwise inhibiting glucocorticoid receptors over an extended period of time where activation of the hypothalamic-pituitary-adrenal axis is preferably minimized.

However, given the exigencies of life, transient periods of stress, depression and pseudo-Cushing's Syndrome can cause a subject's urinary free cortisol to increase for a limited period of time. Thus, it is within the scope of the present disclosure to accommodate urinary free cortisol levels preferably less than, or equal to, twice normal, but no more than three times the upper limit of normal. In fact, up to three times normal urinary free cortisol is what is observed in pseudo-Cushing's Syndrome.

It is, moreover, described a method of treating insulin sensitivity in a patient by at least partially blocking glucocorticoid receptors in tissues responsible for glucose and fat metabolism.

It is, moreover, described a method of improving insulin sensitivity in people having normal cortisol regulation.

It is further described a method of improving insulin sensitivity by administering low doses of the GR antagonist more than once per day to improve insulin sensitivity without inducing hypercortisolism.
Additionally, an object of the present disclosure is to improve insulin sensitivity in hepatic and adipose tissue, in particular.

It is further described to use assays for measuring urine cortisol levels or other surrogate biomarkers of cortisol to determine if dosages of administered GR antagonists and/or inhibitors need to be adjusted downward to prevent 24-hour serum or urine cortisol levels from exceeding about two to three times the upper limit of normal, respectively.

It is, moreover, described a method of improving insulin resistance in humans in need thereof under non-rescue conditions.

The above objects and others discussed below are provided by a method of ameliorating an insulin insensitivity state in a human patient, which entails administering an amount of glucocorticoid receptor antagonist, which amount maintains 24-hour urine or serum cortisol levels within the normal range or up to three or two-fold normal, respectively, in the human patient.

### BRIEF DESCRIPTION OF THE DRAWINGS

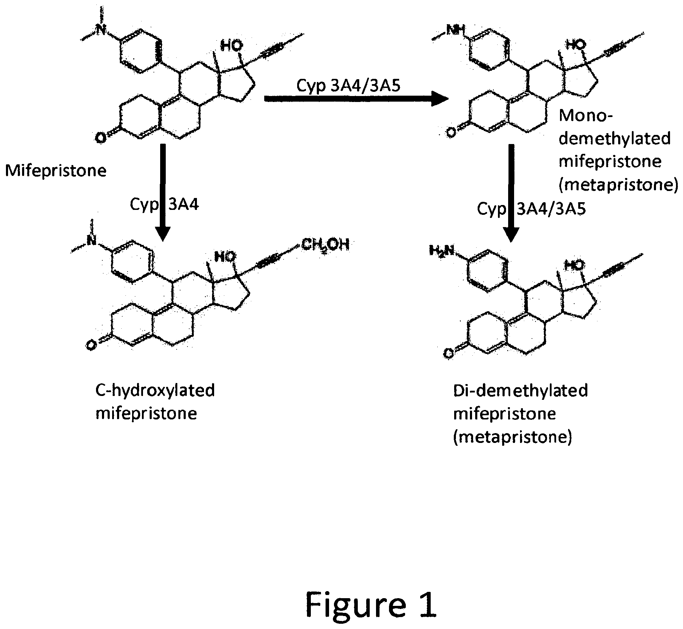

Figure 1 illustrates the major metabolic pathways of mifepristone *in vivo,* including Mono-demethylated mifepristone (metapristone), C-hydroxylated mifepristone, and Di-demethylated mifepristone, these metabolites being formed by oxidation as shown in this figure by cytochromes P450 3A4 (CYP3A4) and P450 3A5 (CYP3A5).

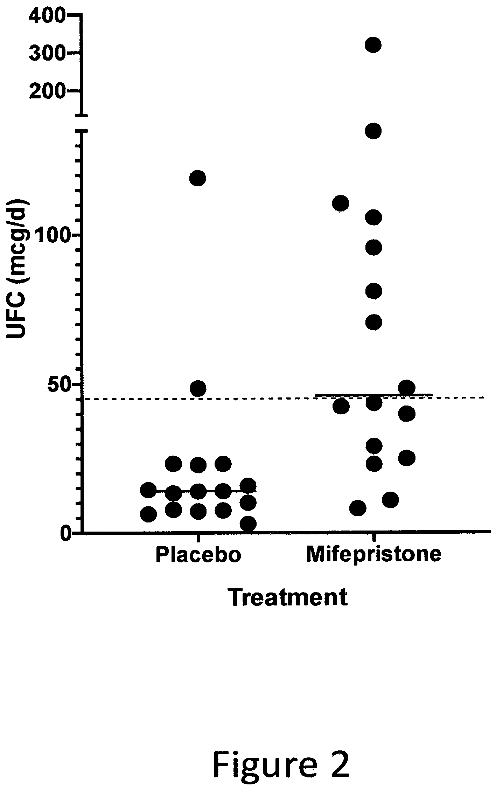

Figure 2 illustrates the urine cortisol excretion over 24 hours at baseline before administration of study agent, and at the end of administration of a placebo or mifepristone (at a dose of 50 mg every six hours for nine days). The upper limit of the normal reference range is 45 micrograms/day. The dots show UFC (urinary free cortisol) during placebo or mifepristone administration. The solid line shows median values and the dotted line represents the upper limit of normal (45 mcg/day).
Figure 3 is a flow diagram of participants throughout a study described herein.
Figure 4 illustrates a 24-hour profile of serum ACTH and cortisol in study participants at the end of either placebo or mifepristone administration.
Figure 5 illustrates time profiles of glucose and insulin following an oral glucose tolerance test (OGTT) in study participants at the end of either placebo or mifepristone administration.
Figure 6 illustrates the effects of placebo and mifepristone on indices of insulin insensitivity.
Figure 7 illustrates the effects of placebo and mifepristone on insulin secretory rates and insulin clearance.
Figure 8 illustrates the effects of placebo and mifepristone on pancreatic B-cell responsivity indices.

### DETAILED DESCRIPTION OF THE INVENTION AND EMBODIMENTS

The present invention is based, at least in part, on the discovery that administration of mifepristone to a human patient in specific dosage ranges and/or at specific times of the day will reverse elements of the metabolic syndrome.

The present invention is also based, at least in part, upon the discovery that administration of mifepristone s to a human patient in specific dosage ranges and/or at specific times of the day will facilitate weight loss.

The present invention is also based, at least, in part, upon the discovery that administration of a GR antagonist, that is mifepristone, in low doses and more than once per day improves insulin sensitivity without inducing biochemical hypercorticolism in humans. The present invention may be viewed conversely as decreasing insulin resistance in humans in need thereof.
It is herein described a method of improving insulin sensitivity by administering an amount of the glucocorticoid receptor antagonist to a human patient in an amount that does not increase mean 24-hour serum cortisol or 24-hour urine cortisol levels more than about two or three times the upper limit of the normal range, respectively, over an extended period of time, such as several weeks or several months.

The present disclosure also provides a method for at least partially blocking, in a human patient, glucocorticoid receptors in tissues that are responsible for glucose and fat metabolism, and to do so over an extended period of time without stimulating the hypothalamic-pituitary-adrenal axis, or with stimulation of 24-hour serum or urine cortisol no more than two or three times the upper limit of normal, respectively.

### Term Definitions

The following terms used throughout the present specification are defined as follows:
**About:** means plus or minus 10% of any value, number or quantity for which the term is applied. For example, as defined here, the phrase "from about 10 to 100" means from 9 to 110. Thus, term "about" as defined herein specifically applies to ranges of values, numbers or quantities.
**Mifepristone:** is 11beta-[p-(dimethylamino)phenyl]-17alpha-(1-propynl)estra-4,9-dien-17beta-ol-3-one, also known as RU-486 or RU-38486. The term "analog" of mifepristone is defined herein as substituents at the 11 and/or 17-positions of the mifepristone ring system that increase GR antagonism by increased selectivity for the GR receptors. Examples of such substituents are the addition of a t-butyl group to the acetylenic group at C-17, or one or more fluorine substituents on the dimethylamino group at C-11, or replacement of the dimethylamino group on the phenyl ring at C-11 with heterocyclic rings, including, for example, morpholine, azolidine, oxacyclobutanes which are optionally methyl-substituted at the steroid ring attachment position and thiomorpholine or sulfone thiomorpholine. Further, the analogs of mifepristone as defined in the present invention satisfy the following conditions: GR antagonism: IC₅₀ (nM); < 20; GR agonism: BC₅₀ (nM)[Emax]: > 2,500; and AR agonism: BC₅₀ (nM) [Emax] > 2,500, using luciferase reporter assays. See Rew Y et al. J. Med. Chem. 2018; 61, 7767-7784,
**Antiglucocorticoids or glucocorticoid antagonists:** means molecules or compounds that reduce the action of glucocorticoids at their receptors by blocking those receptors with the effect of inhibiting, preventing, blockading or antagonizing glucocorticoid action. Glucocorticoid receptor antagonists are also referred to as GR antagonists or glucocorticoid (GR) inhibitors or blockers.GR antagonists are also referred to herein as GRAs.
**Normal blood glucose level:** means a tested blood glucose level that is recognized as normal. For example, with the random blood sugar test, a random blood sugar level of 200 mg per deciliter (mg/dl) or higher suggests diabetes. With the fasting blood sugar test, the following ranges apply: less than 100 mg/dl-normal, from 100-125 mg/dl-prediabetic, and 126 mg/dl or higher on two separate tests-diabetic. With the oral glucose tolerance test (OGTT), the following ranges apply for the 120 minute blood glucose level: less than 140 mg/dl-normal, from 140-199 mg/dl-prediabetic, and over 200 mg/dl diabetic. All of these tests are well known, standard and used routinely. Also in research use is the frequently sampled intravenous glucose tolerance test (FSIVGTT).
**HPA axis:** means hypothalamic-pituitary-adrenal axis.
**Pseudo-Cushing's Syndrome:** is a medical condition in which patients display some of the signs, symptoms and abnormal hormone levels seen in Cushing's Syndrome. Pseudo-Cushing's Syndrome is caused by a physiologic activation of the HPA axis and not by a neoplastic etiology that activates the HPA axis in Cushing's Syndrome or sub-clinical Cushing's Syndrome in which cortisol secretion is dysregulated.
**Pathologic activation of the HPA axis:** This includes neoplastic conditions that produce excess cortisol levels. These conditions include Cushing's syndrome, which has a number of clinical features associated with the cortisol excess. Cushing's syndrome is caused by excess ACTH secretion from a corticotrope tumor or an ectopic ACTH-producing tumor, or from hyperplastic corticotrope cells under the stimulation of ectopic tumoral production of CRH; as well as primary adrenal disorders in which excessive amounts of cortisol secretion cause clinical Cushing's syndrome, such as primary pigmented adrenal nodular disease, macronodular adrenal hyperplasia, or other nodular disorders, for example, those seen in association with McCune Albright Syndrome. Autonomously functioning adrenal nodules, or other adrenal causes of Cushing's syndrome, may display dysregulated cortisol secretion that is insufficient to cause overt Cushing's syndrome; this has been termed "sub-clinical" Cushing's syndrome and may progress to clear-cut cortisol excess and Cushing's syndrome.
**Urine cortisol and other surrogate biochemical markers of HPA activation:** This includes all tests currently available or subsequently developed that may be used to evaluate the effects of GRA dose and schedule on the HPA axis. Normal 24-hour urine free cortisol (UFC) levels range from 39 - 50 mcg/d when measured in most tandem mass spectrometry assays. Values are higher when measured by immunoassays because of cross-reactivity with the antibodies used in those assays with other steroids that are structurally similar to cortisol. Measurement of UFC by tandem mass spectrometry is the preferred method to assess the effect of GRAs on the HPA axis for three reasons: 1. ease of collection, 2. specificity of the assay (it measures only cortisol) and 3. the well-established range of values seen in physiologic stress, or Pseudo-Cushing's syndrome, which is 1 - 3 times the upper limit of the normal reference range. However, other biochemical tests may be used as surrogate biochemical markers of the HPA axis, including, but not limited to measurement of serum or salivary cortisol at multiple time-points throughout a 24 hour period, including both the day and night, which is expressed as either the mean value or the area under the curve. Normal ranges for all cortisol assays and 24-hour assessments will vary depending on the assay technique, and reagents, and must be used based on locally-determined values, or values provided by the manufacturer of an assay kit. The results obtained from any of these assays are then used to determine whether the dosage of the one or more GR antagonists need to be adjusted upwards or downwards so as to maintain 24-hour serum or urinary free cortisol levels at no more than about two or three times the upper level of the normal range, respectively.
**Insulin Insensitivity Disorder:** means any disease or condition that is associated with insulin insensitivity, which includes, for example, diabetes, prediabetes, obesity, lipodystrophy, hypertriglyceridemia, and/or growth hormone excess states.
**Non-rescue conditions:** as used herein means treatment not conducted for the purpose of alleviating the side effects of a medication, or from cessation of a physiologic condition or from pathological causes of hypercortisolism, such as Cushing's Syndrome (see Pathologic activation of the HPA axis, above). As examples, the GR antagonist is employed to improve insulin sensitivity, and not to provide rescue from the effects of anti-psychotic medications, such as weight gain, or to provide rescue from the effects of cessation of exercise and/or caloric restriction, such as adipose tissue mass gain, or to provide rescue from cancers, such as prostate cancer, in the form of treatment for those cancers. The term "non-rescue conditions" is also defined herein to exclude use of GR antagonists, including mifepristone, in patients with excessive or dysregulated cortisol secretion, such as those with Cushing's Syndrome or sub-clinical Cushing's Syndrome due to a functioning cortisol-producing adrenal adenoma. In contrast, the present invention involves the use of mifepristone, to improve insulin sensitivity in people with normal cortisol regulation and under non-pathological conditions. However, the present invention explicitly includes treatment of Pseudo-Cushing's Syndrome, which can be diagnostically distinguished from Cushing's Syndrome. For example, the Dex-CRH test as well as a single measurement of cortisol in serum or saliva at bedtime has demonstrated high diagnostic accuracy in differentiating Pseudo-Cushing's Syndrome from Cushing's Syndrome. The Dex-CRH test is based on the fact that patients with Cushing's Syndrome are still able to mount a pituitary-adrenal response after administration of exogenous CRH despite pre-treatment with low-dose dexamethasone. Patients with Pseudo-Cushing's Syndrome, however, are relatively unresponsive to CRH stimulation when suppressed by dexamethasone. See Alwani et al. European Journal of Endocrinology (2014) 170, 477-486.
**Improve Insulin Sensitivity:** as used herein means a reduction in insulin resistance and, thus, an improvement in how responsive human cells are to insulin. High insulin sensitivity allows the cells of the human body to use blood glucose more effectively to thus maintain a normal blood sugar. This improvement represents a reduced risk of many diseases, including type 2 diabetes. In contrast, the present invention does not seek to merely preserve insulin sensitivity, but rather to improve or alleviate insulin resistance. Consequently, the present invention may be readily distinguished from rescue methodologies that merely seek to prevent adipose rebound and insulin resistance without a need to improve impaired insulin sensitivity. See the definition of "non-rescue" conditions above. In contrast, the present invention improves insulin sensitivity under non-rescue conditions. For example, dysregulated cortisol secretion is an example of a rescue condition. The present invention improves insulin sensitivity in people with normal cortisol regulation.
**Selective GR antagonism:** means little or no activity, agonistic or antagonistic, for other receptors, such as androgen receptors.
**Pure GR antagonism:** as used herein means a minimal level of GR agonism *in vivo.*
**GR antagonist daily dosage:** means about 10 to 75 mg total of one or more GR antagonists per single dosage with more than one dose per day. Generally, two or more, and preferably three of more, dosages are administered daily, about every 4-8 hours. For example, a dosage regimen might entail administration of a 50 mg dosage every 6 hours with a total of 200 mg of one of more GR antagonists administered daily. Further, in normal people, higher doses of mifepristone can be associated with adverse events, such as rash and activation of the hypothalamic-pituitary-adrenal axis, hence a low dose-multiple administration per day regimen, such as a 50 mg dosage every 6 hours, is preferred.
**First Pass Effect:** is the effect observed when mifepristone or another steroidal GR antagonist is taken orally. The mifepristone or other GR antagonist passes from the gut to the liver where the steroidal GR antagonist is metabolized. Thereby, the adipose tissue in the omentum and the liver "see" the highest concentration of the GR antagonist. Once past the hepatic metabolism, the systemic concentration of the steroidal GR antagonist is reduced and, thus, the hypothalamus and pituitary "see" these lower concentrations. Thus, a first pass effect is believed to be responsible for the reduced **sensitivity** of the dose-response curve of the hypothalamic-pituitary-adrenal axis compared to that for adipose tissue and the liver. However, the inventors are not bound by any theory in general, and do not intend for the invention disclosed herein to be so bound. Nonetheless, two important aspects of the invention disclosed herein are: 1) hepatic and adipose tissue insulin **sensitivities** are beneficially affected, and 2) HPA axis activation is minimized. These two important aspects of the disclosed invention are accentuated by use of low dose-multiple daily administration of one or more GR antagonists with an upper limit on total daily dosage.

### Consideration of the mode of administration, dose and schedule of administration of GR antagonists

The present inventors have discovered that administration of mifepristone, either orally, by depot or transdermally (by patch, for example) to patients in specific dosages or dosage ranges and/or times of the day might reverse elements of the metabolic syndrome.

Furthermore, the inventors considered that activation of the hypothalamic-pituitary-adrenal axis might override the effects of glucocorticoid antagonism. This would occur at antiglucocorticoid doses or schedules of administration that antagonize cortisol's inhibitory effect on ACTH secretion, leading to an increase in ACTH and cortisol, and potentially diminishing or abolishing an effect on insulin insensitivity should systemic levels of GR antagonist not be sufficient to block the effects of increased cortisol. Thus, the present inventors considered that the dosing regimens should be selected that differentially block cortisol action at peripheral levels, but which do not activate the hypothalamic-pituitary-adrenal axis to an extent that would override the peripheral blockade.

The present inventors have, in fact, discovered that mifepristone may be used advantageously to improve insulin sensitivity.

Generally, dosages of one or more glucocorticoid antagonists are administered in the amount of from about 500 micrograms (0.5 mg) to about 100 mg once or more per day. For example, 50 mg of mifepristone may be administered every six (6) hours to a patient having abnormal glucose tolerance, thereby improving insulin sensitivity. The treatment regime is adjusted until the patient's blood glucose level attains and maintains a normal level as measured by a standard known glucose test. A treating physician may, of course, reduce or increase dosages as deemed advisable and routinely monitor the effectiveness of dosages.

Generally, the same dosage range may be used for any one or more GR antagonists when used in accordance with the present invention to increase insulin sensitivity.

### Testing for Cortisol Levels to Evaluate the Need for Adjustment of Dosage of One or More GR Antagonists for Sequent Administration of the One or More GR Antagonists

In accordance with another aspect of the present disclosure, insulin, glucose and 24-hour serum or urine cortisol levels are measured once steady-state blood levels are reached, at five or more half-times of the clearance of one or more GR antagonists to determine a more preferred dosage of the one or more GR antagonists for sequent administration. It is considered that if the dosage of the one or more GR antagonists is too low, insulin sensitivity will not be improved, whereas if the dosage thereof is too high the hypothalamus-pituitary-adrenal (HPA) axis will be activated, and too much cortisol will be produced. To achieve maximum dose efficiency, insulin, glucose and cortisol testing should be conducted prior to dose adjustments for sequent administration of the one or more GR antagonists to improve insulin sensitivity without activating the HPA.

Generally, whether steroidal or non-steroidal GR antagonists are used, the applicable dosages range from 10 to 75 mg in total per dose with doses being administered from 1 to 6 times per day. The treating physician may adjust the number of doses per day as deemed required based upon measured serum, glucose, insulin and cortisol levels.

The present invention uses dosages of from about 30 to 70 mg per dose of the GR antagonist with administration of 3 to 5 times daily. For example, 50 mg of mifepristone can be administered every 6 hours with 200 mg being the total daily dose. Generally, the total daily dose of one or more GR antagonists will not exceed about 500 mg daily in total.

Although any mode of administration may be used for the one or more GR antagonists, such as oral, intravenous and/or intramuscular injection or topical, oral administration is preferred.

The following examples are provided only for purposes of illustration and are not intended to be limitative.

### Example 1:

A randomized, placebo-controlled, double-blind crossover trial was conducted to evaluate the use of an anti-glucocorticoid as a means for ameliorating insulin resistance. Mifepristone, 50 mg, or a placebo, was administered every six hours for nine days to sixteen overweight or obese (BMI 25-37 kg/m²) subjects (seven women). Participant ages ranged from 48 to 64 years. Ten had prediabetes and six had mild diabetes type II (HgbA1c less than 7%). Treatment of the glucose disorder with diet or a stable dose of metformin (used by two patients) was maintained for three (3) months before the study. The interventions were nine days of mifepristone or placebo treatment followed by a six (6) to eight (8) week wash out and cross over to the other arm. At baseline and at the end of each treatment period, parameters of insulin sensitivity were assessed by a frequently sampled intravenous glucose tolerance test (FSIVGTT) and an oral glucose tolerance test (OGTT). A whole body glucose disposal index of insulin sensitivity (S_{I}) was derived using a minimal model analysis. Adipose tissueS_{I}, which reflects insulin-induced free fatty acid suppression, was estimated by the log-linear slope of free fatty acid levels and the AUC of insulin during the FSIVGTT. Matsuda index, oral glucose insulin sensitivity index (OGIS, ml/min/m²), and hepatic insulin resistance index (HIRI, mg/dl x microU/ml x 10⁵) were derived from OGTT. Adipo-IR, a surrogate measure of adipocyte insulin resistance, was calculated by multiplying fasting concentrations of free fatty acids and insulin (mmol x pmol/L). Comparison of various post-treatment parameters was performed using crossover ANOVA. This analysis takes into account specific treatment arm, treatment order, and treatment effects. Thus, carryover effects were explicitly tested for in the crossover study.

The glucocorticoid receptor blockade did not affect body weight (p=0.59). Fifteen subjects had urine free cortisol values less than three-times normal, and eight of these were within the normal range. One subject had elevated values at all time-points before and during both mifepristone and placebo. Mifepristone administration for one week did not alter whole body glucose disposal indices of insulin sensitivity (FSIVGTT-S_{I}: p=0.60, Matsuda Index: p=0.16, and OGIS: p=0.92). However, glucocorticoid receptor blockade significantly decreased fasting insulin levels when compared with placebo (mean + SD; 95.7 + 76.1 vs 142.8 + 102.2 pmol/L, p = 0.0006), as well as fasting glucose levels (mean + SD, 100.4 + 15.3 vs 107.8 + 15.7 mg/dL). Glucocorticoid receptor blockade also reduced HIRI (70+44.3 vs 50.2 + 38.7, p = 0.08) and Adipo-IR (65.5 + 43.8 vs 50.0 + 46.0, p < 0.001) and improved Adipose SI (42.8 + 23.9 vs 61.7 + 32.9, p < 0.001).

Thus, in patients with abnormal glucose tolerance, short term glucocorticoid receptor blockade improves hepatic and adipose tissue insulin sensitivity without significantly altering whole body sensitivity. This result also is important for the additional reason that insulin resistance in individual tissues can cause reduced skeletal muscle metabolic flexibility.
Examples 1 and 2 are from the same clinical protocol, however Example 2 provides additional data and analysis thereof that became available after the initial data of Example 1 were processed and analyzed.

### Example 2:

To further demonstrate the effectiveness of the present invention in improving insulin sensitivity under non-rescue conditions with patients having normal cortisol dynamics, the following randomized, triple-blinded, placebo-controlled, cross-over study was conducted using mifepristone as an exemplary and representative GR antagonist.

The inventors conducted a random, triple-blinded, placebo-controlled, cross-over study using mifepristone (50 mg every 6 hours, 200 mg total daily dose) in overweight/obese individuals (n = 16, 44% female) with pre-DM (pre-diabetes mellitus) or mild type 2 diabetes mellitus (glycated hemoglobin A1C greater or equal to 7). Mifepristone or placebo was administered for 9 days followed by a washout period of 6 to 8 weeks, and then crossover to the other treatment arm. At baseline and after each treatment period, the oral glucose tolerance test and frequently sampled intravenous glucose tolerance test were performed. Insulin sensitivity (S_{I}) was measured using Matsuda index and oral glucose insulin sensitivity index (OGISI). Hepatic S_{I} was assessed using hepatic insulin resistance index (HIRI), and adipose tissue S_{I} was assessed using adipo-IR adipose tissue insulin sensitivity index (ATISI).

### Methods:

### Trial Design:

The study in this example used a prospective, randomized, double-blind placebo-controlled, cross-over design in which eligible subjects were randomized in blocks of six to the initial arm of the study: either placebo or mifepristone (50 mg tablets every 6 hours) for 9 days. This was followed by a 6-8 week washout period. Subjects were then crossed over to the other treatment arm for an additional 9 days. The study was conducted at the National Institutes of Health (NIH) Clinical Center, Bethesda, Maryland, U.S.

### Participants:

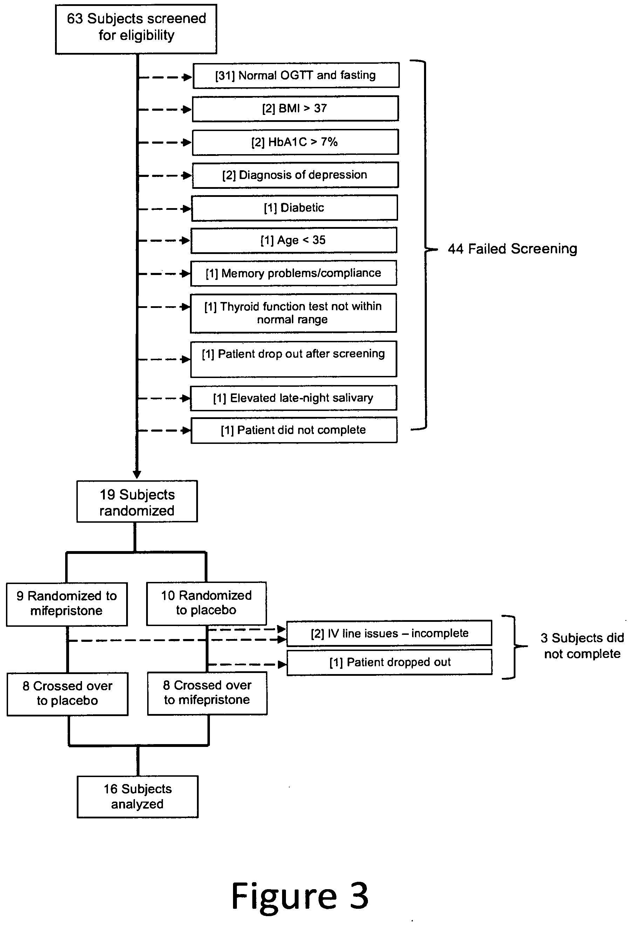

Subjects eligible for this study included men and women 35-70 years of age who were overweight or obese (BMI 25-37 kg/m²) with pre-diabetes (defined as fasting glucose of 100 to 125 mg/dL or a 2-hour postprandial glucose value of greater than or equal to 140 mg/dL and less than 200mg/dL during an oral glucose tolerance test (OGTT) or mild diabetes (defined as a HgbA1C greater than 6.5% to less than or equal to 7%). Eligible subjects took no medications or were on a stable dose of metformin and no other hypoglycemic agents for at least 3 months before beginning the study. The study excluded pregnant and lactating women and subjects with uncontrolled hypertension (> 180/110 mmHg) or any unstable medical conditions (e.g., impaired cardiac function, severe respiratory insufficiency), liver function tests (ALT and AST) more than 3 times the normal upper limit, severe renal impairment (creatinine clearance < 30 ml/min), evidence of HIV or hepatitis C infection, history of hemorrhagic disorders or use of anticoagulants, history of endometrial cancer or hyperplasia, unexplained vaginal bleeding or endometrial thickness greater than 6 mm, changes in lipid lowering medication dose within 1 month of study entry, use of any medications or dietary supplements with strong inhibition of CYP3A4, use of grapefruit juice or herbal supplements within 14 days of study drug initiation, use of glucocorticoids or megestrol within 6 months of study initiation, use of estrogen-based hormone therapy, depression (evaluated by PHQ-9 with a cut off of greater or equal to 10), excessive alcohol use (> 2 drinks per day for women and > 3 drinks per day for men), untreated thyroid dysfunction, moderate/severe anemia (hemoglobin < 10g/dL), blood donations totaling 500 mL or more within one month of study initiation, prolonged QTc interval, and subjects in a weight loss program or who were actively dieting. Perimenopausal and postmenopausal women underwent a transvaginal ultrasound to evaluate endometrial thickness, and a pelvic MRI was performed if the transvaginal ultrasound did not provide an accurate reading of the endometrial thickness. See Figure 3, which provides a flow diagram of participants throughout the study. Subjects were required to be weight-stable for the 2 weeks prior to entry and not to start any new diet/exercise programs during the study.

Study subjects were screened and evaluated at the NIH Clinical Center for eligibility. 63 subjects were consented and screened for eligibility for the study protocol, with 44 subjects not studied based on inclusion and exclusion criteria. The remaining 19 subjects were initially enrolled in the study, with a final sample of 16 subjects completing the study with sufficient data for analysis.

### Study Approval:

The study was approved by the Institutional Review Board of the National Institute of Child Health and Human Development, and all procedures were performed as per the institutional guidelines. Eligible participants provided written informed consent to participate in protocol 11-CH-0208 (Clinicaltrials.gov; NCT01419535).

### Sample Size:

Patient recruitment and assessment for eligibility is shown in Figure 3. Initially, 19 subjects underwent randomization. One subject dropped out, while 2 other subjects had malfunctioning intravenous access resulting in incomplete data. The final analysis was performed on 16 subjects. Though the protocol listed a sample size goal of 17 subjects, analysis for 16 subjects was used. Beyond this, there were no changes to the protocol methodology after trial commencement. An interim analysis was conducted to evaluate the effect of the mifepristone dose (50 mg/6 hr) on the hypothalamic-pituitary-adrenal axis. The study statistician was unblinded to the first 6 subjects (one randomization block) to evaluate whether the 24-hour UFC levels following mifepristone treatment were equal to, or more than twice, the upper limit of the reference range in 4 or more of the 6 subjects. In that scenario, the mifepristone dose would be decreased to 25 mg every 6 hours (100 mg/day). However, UFC values remained below the threshold and no dose adjustment was required.

### Randomization, blinding and study drug management:

Mifepristone was obtained through an active IND under a CRADA agreement partnership with Laboratoire HRA-Pharma (Paris, France). Mifepristone and inert, look-alike placebo were packaged into 50 mg capsules by the NIH Clinical Center Pharmaceutical Development Service.

### Procedures of the study

To reduce potential variability associated with estrogen in premenopausal women, testing was performed in the early follicular phase (days 1-7 of menstrual cycle). Baseline height and weight were measured after an overnight fast. Subjects received an isocaloric regular diet during the inpatient part of the study, and those on metformin took the same dose throughout the study. Baseline testing took place took place over three inpatient days at the NIH Clinical Center, during which fasting blood was drawn, 24-hour urine was collected, 24-hour serial blood draws were obtained, and patients received both a frequently sampled glucose tolerance test (FSIVGTT) and a 75-g oral glucose tolerance test (OGTT). Subjects were then randomized to receive placebo or mifepristone for 9 days, of which the first 6 days the subjects returned home, and the final 3 days subjects underwent the same metabolic testing as during the baseline period. Subjects randomized to the mifepristone treatment arm were instructed to take one capsule (50 mg) every 6 hours (200 mg/day) over the course of 9 days. The placebo treatment arm received the same instructions to take one capsule every 6 hours over the course of the 9-day treatment period. Following the completion of the post-drug testing, subjects entered a 6-8 week washout period, after which they were crossed over to the other treatment arm, serving as their own controls for the study drug treatments.

All subjects were instructed to record any adverse effects and to report them to study personnel, and women were advised to keep a record of any abnormal menstrual bleeding. For proper safety monitoring, subjects returned approximately one week (day 19) and three weeks (day 33) after the discontinuation of the study drug for both treatment arms. During this safety visit, serum electrolytes, renal and liver function, and complete blood count were measured, as well as evaluation and treatment for potential treatment-related abnormalities/adverse events.

### Study Interventions

### Frequently Sampled Intravenous Glucose Tolerance Test:

Insulin sensitivity index, S_{I}, was determined from the minimal model using data from the insulin-modified FSIVGTT as previously described (version 6.02; MinMOD Millenium, Los Angeles, CA). Briefly, after an overnight fast, on day 2 of baseline and post-treatment testing, two intravenous catheters were placed, one in each arm or in the antecubital veins. A 50% dextrose (0.3gm/kg) bolus was infused intravenously over 1 minute at timepoint 0-min, and an insulin (0.03 units/kg) bolus was infused at the 20-min timepoint. Several blood draws were obtained at: -10, -1, 0, 1, 2, 3, 4, 5, 6, 7, 8, 9, 10, 12, 14, 16, 19, 22, 23, 24, 25, 27, 30, 40, 50, 60, 70, 80, 90, 100, 120, 150 and 180 minutes for measurement of glucose, insulin and free fatty acids.

### Oral Glucose Tolerance Test:

On day 3 of baseline and post-treatment testing, subjects underwent a 75-gram OGTT. Serial blood draws for measurement of glucose, insulin and c-peptide were obtained at -5, 0, 30, 60, 90, 120, 150, and 180 minutes.

### Study Endpoints

### Surrogate Measures of Insulin Sensitivity and ß-Cell Function

The Matsuda index and Oral Glucose Insulin Sensitivity Index (OGIS), surrogate indices of whole-body insulin sensitivity were derived from plasma glucose and insulin concentrations during the OGTT as previously described. Abdul-Ghani MA, et. al., Diabetes Care 2007; 30 (1): 89-94. HIRI was calculated as the product of the area under the curves of glucose and insulin for the first 30 minutes of the OGTT. Abdul-Ghani MA, *ibid.* The Adipo-IR index, a surrogate measure for fasting adipose adipose-tissue insulin resistance, was calculated as the product of fasting insulin and fasting FFA. Sondergaard E. et al., J. Clin. Endocrinol. Metab. 2017; 102(4):1193-9. A dynamic surrogate index of adipose tissue insulin sensitivity was assessed by calculating insulin's ability to suppress FFA levels during a FSIVGTT. Carpenter A. et al., Diabetes 2002; 51(6):1867-75 and Trottier A. et al. Fertil. Steril. 2992; 98 (6):1627-34. The adipose tissue insulin sensitivity index was calculated as the ratio of the slope of the linear decrease in natural log transformed FFA [Ln (FFA) slope] during the first 90 minutes of the FSIVGTT and the AUC of insulin during that 90-minute period (AUC _{Insulin 0-90 min}). Mathematical modeling of C-peptide concentrations was used to estimate insulin secretory rates and calculate ß-cell responsivity parameters (basal (ØB); first-phase (Ø1); second phase (Ø2); and total (ØTOT) as previously described. Toffolo G. et al., Metabolism 1999; 48 (9):1162-6 and Cobelli C. et al. Am. J. Physiol. Endocrinol. Metab. 2007; 293 (1): E1-E5. Assuming an absence of hepatic C-peptide extraction, postprandial insulin clearance was calculated as the ratio of mean insulin secretion and insulin concentration. Trico D. et al. JCI Insight 2018; 3(24):e124912.

### Statistical Analysis:

Changes in insulin sensitivity as measured by FSIVGTT were prospectively designated as the primary endpoint for this study. All other comparisons were considered secondary. Continuous variables were described as mean and standard deviation and categorical data were represented as proportions and frequencies. All continuous data were examined for normality and homogeneity of variances and were either log transformed as appropriate or assessed using non-parametric tests. Between and within treatment comparisons were performed using mixed models for a crossover design that also considers sequence effect, if any. Treatment-by-period interaction was tested for primary outcome. Two-sided p-values were used for all analyses. Repeated measures were analyzed by repeated-measure ANOVA including treatment group time, and treatment-time interaction as factors. Post-hoc pairwise comparisons were performed by Bonferroni's test. The level of statistical significance was set *a priori* at < 0.05 and the p-values were not adjusted for multiple comparisons. Data were analyzed with JMP version 13.0 (SAS Institute, Cary, NC) and GraphPad Prism 7 (GraphPad Software Inc., La Jolla, CA).

### Results:

There were no significant differences observed in total body insulin sensitivity after mifepristone treatment. However, the AUC for insulin was significantly lower after mifepristone treatment compared to placebo. Mifepristone treatment resulted in significant improvement in adipose tissue sensitivity, and there was a near-significant improvement in hepatic sensitivity. The conclusion is that short-term GR antagonist use, as exemplified by mifepristone, improves adipose and hepatic insulin sensitivity among overweight or obese individuals with hyperglycemia without inducing hypercortisolism.

### Baseline Characteristics of Study Subjects:

The baseline characteristics of the 16 subjects who completed the study are described in table 1 below.

**Table 1: Clinical and Metabolic Characteristics of Subjects at Baseline: n =16**

| | |
|---|---|
| Age (years) | 55.5 ± 7.9 |
| Sex (F/M) | 7/9 |
| Weight (kg) | 97.1 ± 15.6 |
| Body-Mass Index (kg/m3) | 32.2 ± 3.7 |
| Systolic Blood Pressure (mmHg) | 121 ± 17 |
| Diastolic Blood Pressure (mmHg) | 69 ± 10 |
| Total Cholesterol (mg/dL) | 181.1 ± 52.4 |
| HDL Cholesterol (mg/dL) | 44.7 ± 9.6 |
| LDL Cholesterol (mg/dL) | 107.8 ± 39.3 |
| Triglycerides (mg/dL) | 142.6 ± 83.8 |
| Free Fatty Acids (mmol/L) | 0.613 ± 0.195 |
| Fasting Glucose (mg/dL) | 102.7 ± 11.6 |
| Fasting Insulin (pmol/L) | 138.9 ± 89.0 |
| HbA1C (%) | 5.98 ± 0.60 |
| HOMA-IR | 5.16 ± 3.49 |
| QUICKI | 0.310 ± 0.025 |
| OGIS (ml/min/m²) | 283.9 ± 66.3 |
| Matsuda Index | 2.15 ± 1.05 |
| S_{I} (min⁻¹ · µU·ml¹) | 1.06 ± 0.61 |
| Urinary Free Cortisol (µg/24 hrs) | 22.9 ± 17.2 |

For the above listed characteristics, the following is noted. 44% of the participants were female. Participants were overweight or obese with a mean BMI of 32.2 kg/m² (range: 25.7 to 37.5 kg/m²). 63% of the cohort (n = 10) were prediabetic [impaired fasting glucose (IFG), 13% n = 2; impaired glucose tolerance (IGT), 31%, n = 5; and both IFG and IGT, 19%], n = 3, and 37%, n = 6 were diagnosed with T2DM. The average (HgbA1C) (± SD) at baseline of the entire, prediabetic, and diabetic groups was 5.98 ± 0.57%, 5.88 ± 0.43% and 6.26 ± 0.59%, respectively. Two of the diabetic patients were taking stable doses of metformin. The mean systolic and diastolic blood pressures (BP), were 121 ± 17 and 69 ± 10 mm of Hg, respectively. 63% of the participants (n = 10) were on antihypertensive agents and 31% (n = 5) were on statins. 38% of the participants had low levels of HDL cholesterol (< 40 mg/dL (men)or < 50 mg/dL (women)) or high triglyceride (TG) levels (> 150 mg/dL). Surrogate measures of insulin sensitivity indices based on glucose and insulin levels during fasting (quantitative insulin sensitivity check index, QUICKI, and index of homeostasis model assessment, HOMA-IR) and glucose tolerance tests (oral glucose insulin sensitivity index, OGIS and Matsuda index from oral glucose tolerance test (OGTT) and insulin sensitivity index derived from the frequently sampled intravenous glucose tolerance test (FSIVGTT), S_{I}) are reported in Table 1. HOMA-IR > 2.5, QUICKI less than or equal to 0.331, OGIS less than or equal to 344, Matsuda Index less than or equal to 2.5, or S_{I} less than or equal to 2.1 has been used to define insulin resistance. Owei I. et al. BMJ Open Diabetes Res. Care 2017; 5 (1): e000415; Kernan WN et al. Stroke 2013; 34(6):1431-6; Antoniolli LP et al. Diabetol. Metab. Syndr. 2018; 10:65; Ascaso JF et al. Diabetes Care 2003; 26(12):3320-5. Based on the mean values of HOMA-IR, QUICKI, OGIS, Matsuda index and S_{I}, subjects in this cohort were insulin resistant. See Table 1. The mean 24-hour urinary free cortisol (UFC) values were within normal limits (22.9 ± 17.2 µg/24 hr.; normal: 3.5-45 µg/24 hr.) The final study population exhibited the inclusion criteria set for the trial.

### Effects of Mifepristone Treatment on Circulating Levels of Cortisol and ACTH and 24-hour Urinary Free Cortisol:

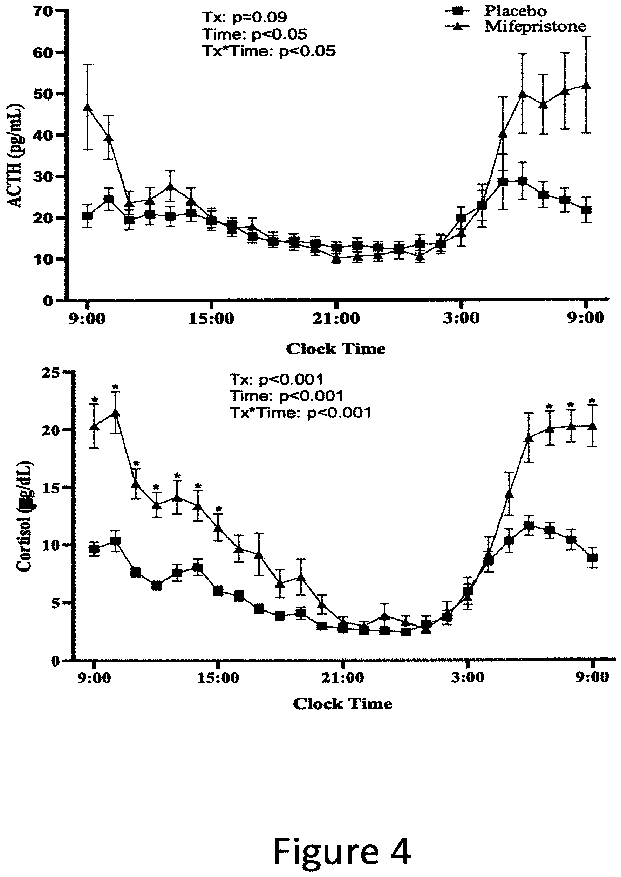

Mifepristone is known to block the negative feedback effects of cortisol, which leads to elevations in ACTH and cortisol, by antagonizing GRs. Gailllard RC et al., Proc Natl Acad Sci U S A. 1984; 81 (12): 3879-82. Mean ACTH and cortisol levels were higher during mifepristone than placebo administration specifically during mornings. See Figure 4. ACTH levels were significantly higher from 5AM-10AM, while the cortisol difference also started around 5AM, the levels remained significantly elevated until about 5PM when compared with placebo. See Figure 4. The area under the curve (AUC) measurements for ACTH and cortisol were significantly higher during mifepristone treatment (574.5 ± 260.8 vs. 448.7 ± 193.5 pg/mL · min for ACTH, p<0.001, and 255.4 ± 61.9 vs. 151.1 ± 21.1 µg/dL·min for cortisol; p<0.001). Overall, mifepristone significantly increased 24-hour UFC compared with placebo (74.1 ± 75.3 vs, 25.3 ± 30.2 µg/24 hr. p=0.0001). These stimulatory effects of mifepristone were not uniform, with post-treatment mean UFC above the normal limit in 8 subjects (120.4 ± 83.9 µg/24 hr). In the mifepristone treatment arm, serum cortisol AUC levels were significantly higher in the subjects with higher 24-hr UFC compared to the subjects with normal 24-hr UFC (284.9 ± 39.9 vs, 229.8 ± 68.5 µg/dL·min for cortisol p=0.009. Plasma DHEA sulfate (DHEAS) and androstenedione levels were significantly higher after mifepristone therapy when compared with placebo. See Table 2 below. Overall, these results indicate that an exemplary dosage of mifepristone (50 mg tablets every 6 hours) for 9 days substantially increased cortisol and ACTH levels as a compensatory response to GR blockade.

**Table 2: Clinical and Metabolic Parameters and Hormone Levels After Placebo and Mifepristone Administration:**

| | **Placebo** | **Mifepristone** | **P value** |
|---|---|---|---|
| Weight (kg) | 97.7 ± 16.3 | 97.4 ± 8 | 0.59 |
| Body-Mass Index (kg/m²) | 32.4 ± 4.3 | 32.2 ± 3.9 | 0.39 |
| Systolic Blood Pressure (mmHg) | 121 ± 12 | 130 ± 13 | 0.009 |
| Diastolic Blood Pressure (mmHg) | 71 ± 9.5 | 77 ± 9.2 | 0.01 |
| Total Cholesterol (mg/dL) | 177.3 ± 38.7 | 163.6 ± 419 | 0.004 |
| HDL Cholesterol (mg/dL) | 45 ± 8.8 | 41.3 ± 11.9 | 0.01 |
| LDL Cholesterol. (mg/dL) | 101.9 ± 31.1 | 97.1 ± 34.6 | 0.10 |
| Triglycerides (mg/dL) | 152 ± 79.3 | 125.6 ± 65.6 | 0.002 |
| Total testosterone (male) (ng/dL) | 355.2 ± 124.1 | 235.2 ± 87.5 | 0.01 |
| Androstenedione (ng/dL) | 66.7 ± 41.7 | 110 ± 64.7 | 0.007 |
| DHEA Sulfate (µg/mL) | 0.82 ± 0.45 | 1.04 ± 0.64 | 0.004 |
| Urinary Free Cortisol (µg/24 hr) | 25.3 ± 30.2 | 74.1 ± 75.3 | 0.0001 |

Post-treatment values shown are unadjusted means ± SD. Post-treatment values were adjusted for baseline value and treatment group using mixed-model regression. P values indicate significance for comparisons between mifepristone and placebo.

Figure 4 shows a serial time course of plasma ACTH (A) and cortisol (C) every hour from 9 AM one day to 9 AM the following day. Data shown are mean ± SEM with p values provided for the effects of treatment group, time, and the treatment-time interaction, which was obtained with repeated-measures ANOVA with post-hoc Bonferroni's test. Tukey box and whisker plots show ACTH (B) and cortisol (D) area under the curve for the 24-hour period. Top and bottom box limits represent the 75^{th} and 25^{th} percentile, respectively; midlines represent the median, and top and bottom whiskers represent the 75^{th} percentile plus 1.5 of the interquartile range (IQR) and the 25^{th} percentile minus 1.5 IQR, respectively. Post-treatment values were adjusted for baseline and treatment group using mixed-model regression. P values indicate significance for comparisons between mifepristone and placebo.

### Effect of Mifepristone Treatment on Body Weight, Blood Pressure (BP) and Serum Lipids:

Changes in body weight, BP and serum lipid concentrations for each study subject were evaluated at baseline and after 9-day administration of mifepristone or placebo. There were no significant differences observed in body weight or BMI between the treatment arms. See Table 2. BP was higher after mifepristone treatment for both systolic and diastolic values. Mifepristone treatment was associated with significantly lower serum total cholesterol, HDL cholesterol and triglycerides. See Table 2. The mean plasma LDL levels were lower after mifepristone treatment, but the difference was not statistically significant.

### Effect of Mifepristone on Plasma Glucose, Serum Insulin and Indices of Insulin Sensitivity:

Mifepristone treatment resulted in significant lowering of plasma glucose and insulin. See Table 3 below.

**Table 3: Effects of Placebo and Mifepristone Administration on Metabolic Parameters and Measures of Insulin Sensitivity and Resistance**

| | **Placebo** | **Mifepristone** | **P value** |
|---|---|---|---|
| Fasting Glucose (mg/dL) | 107.8 ± 15.7 | 100.4 ± 15.3 | <0.001 |
| Fasting Insulin (pmol/L) | 142.8 ± 102.2 | 95.6 ± 76.1 | <0.001 |
| HOMA-IR | 5.78 ± 4.92 | 3.58 ± 3.27 | <0.001 |
| QUICKI | 0.310 ± 0.029 | 0.332 ± 0.034 | <0.001 |
| Si (min⁻¹·µU·ml⁻¹) | 1.41 ± 0.87 | 1.49 ± 1.17 | 0.60 |
| OGIS (ml/min/m²) | 308.9 ± 72.9 | 305 ± 48.9 | 0.92 |
| Matsuda Index | 2.52 ± 1.96 | 2.67 ± 1.03 | 0.16 |
| Hepatic Insulin Resistance Index (HIRI) | 70 ± 44.3 | 50.2 ± 38.7 | 0.08 |
| Adipose-tissue Insulin Resistance Index (Adipo-IR) | 65.5 ± 43.8 | 50.0 ± 46.0 | <0.001 |
| Adipose-tissue Insulin Sensitivity Index | 42.8 ± 23.9 | 61.7 ± 32.9 | <0.001 |

Post-treatment values shown are unadjusted means ± SD. QUICKI, Quantitative Insulin Sensitivity Check Index; HOMA-IR, Homeostatic Model Assessment of Insulin Resistance; OGIS, Oral Glucose Insulin Sensitivity; S_{I}, Insulin Sensitivity Index derived from the insulin-modified frequently sampled intravenous glucose tolerance test. Post-treatment values were adjusted for baseline value and treatment group using mixed-model regression. P values indicate significance for comparisons between mifepristone and placebo.

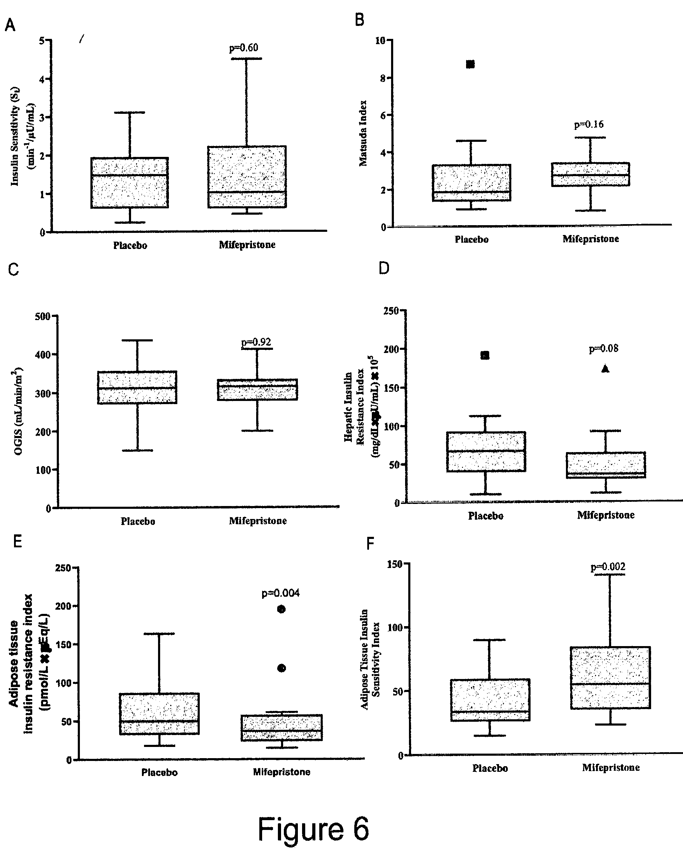

The results shown in Table 3 indicate that mifepristone significantly reduced HOMA-IR and improved QUICKI as compared with placebo. The primary endpoint, insulin sensitivity index (S_{I}) as measured by FSIVGTT was not significantly different between the treatment arms. See Table 3 and Figure 6A. The time course of glucose and insulin concentrations during the OGTT were not significantly different between the treatment groups. See Figures 6A and 6C. The AUC measurements for glucose during OGTT appeared to be higher in the mifepristone group, but this apparent difference was not statistically significant. See Figure 6B. However, AUC for insulin was significantly lower by about 25% during mifepristone treatment as compared to placebo. See Figure 6D. Overall glucose insensitivity indices derived from OGTT, OGIS and Matsuda index were not significantly different between placebo and mifepristone. See Table 3 and Figures 6B and 6C. Hepatic insulin resistance index (HIRI) tended to be lower following mifepristone administration (p=0.08). See Table 3 and Figure 6D. Adipose tissue insulin resistance was assessed during the fasting state (Adipo-IR) and a measure of insulin-induced suppression of free fatty acids (FFA) levels during FSIVGT adipose tissue insulin sensitivity index (Adipo-SI) was estimated by the log-linear slope of FFA levels during FSIVGTT corrected for circulating insulin measured (AUC) during the same period. Mifepristone administration was associated with a significant attenuation in Adipo-IR and increase in Adipo-SI. See Figures 6E and 6F.

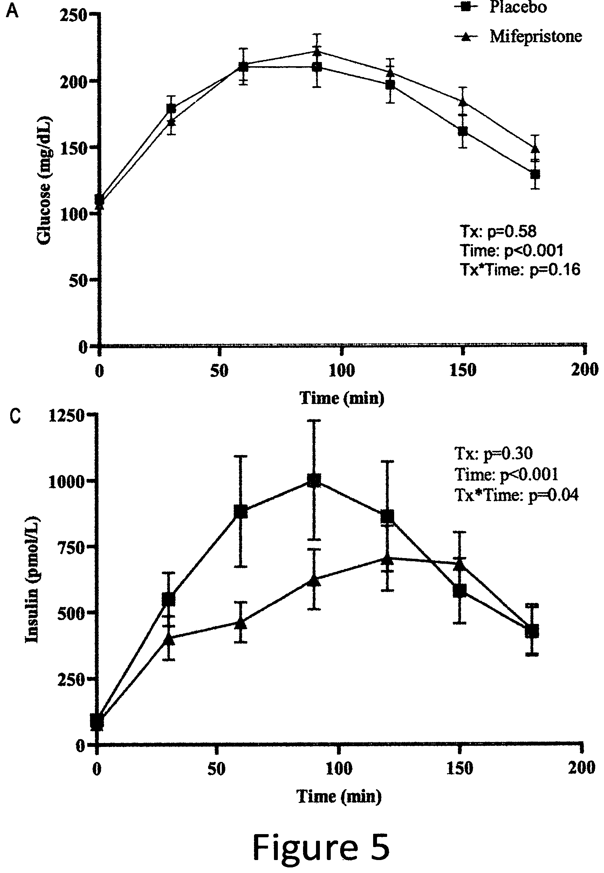

Figure 5 shows the results of a time course of plasma glucose (A) and insulin (C) during OGTT. Data shown are mean ± SEM. Values for the effects of treatment group, time and the treatment-time interaction were obtained with repeated-measures ANOVA with post-hoc Bonferroni's test. Tukey box and whisker plots of glucose (B) and insulin (D) area under the curve for the duration of 3-hr OGTT. Top and bottom box limits represent the 75^{th} and 25^{th} percentile, respectively; midlines represent the median; and top and bottom whiskers represent the 75^{th} percentile plus 1.5 of the interquartile range (IQR) and the 25^{th} percentile minus 1.5 IQR, respectively. Symbols represent values that lie outside the range of the whiskers. Post-treatment values were adjusted for baseline value and treatment group using mixed-model regression. P values indicate significance for comparisons between mifepristone and placebo.

Figure 6 shows the effects of placebo and mifepristone on indices of insulin sensitivity. Tukey box and whisker plots (A) S_{I}, insulin sensitivity index derived from the insulin-modified frequently sampled intravenous glucose tolerance test, (B) Matsuda index, (C) OGIS, oral glucose insulin sensitivity index, (D) hepatic insulin resistance index (HIRI), (E) adipose tissue insulin resistance index, and (F) adipose tissue insulin sensitivity index. Top and bottom box limits represent the 75^{th} and 25^{th} percentile, respectively; midlines represent the median; and top and bottom whiskers represent the 75^{th} percentile plus 1.5 of the interquartile range (IQR) and the 25^{th} percentile minus 1.5 IQR, respectively. Symbols represent values that lie outside the range of the whiskers. Post-treatment values were adjusted for baseline value and treatment group using mixed-model regression. P values indicate significance for comparisons between mifepristone and placebo.

### Effect of Mifepristone on Insulin Secretory Rates, Insulin Clearance and ß-Cell Responsivity Indices:

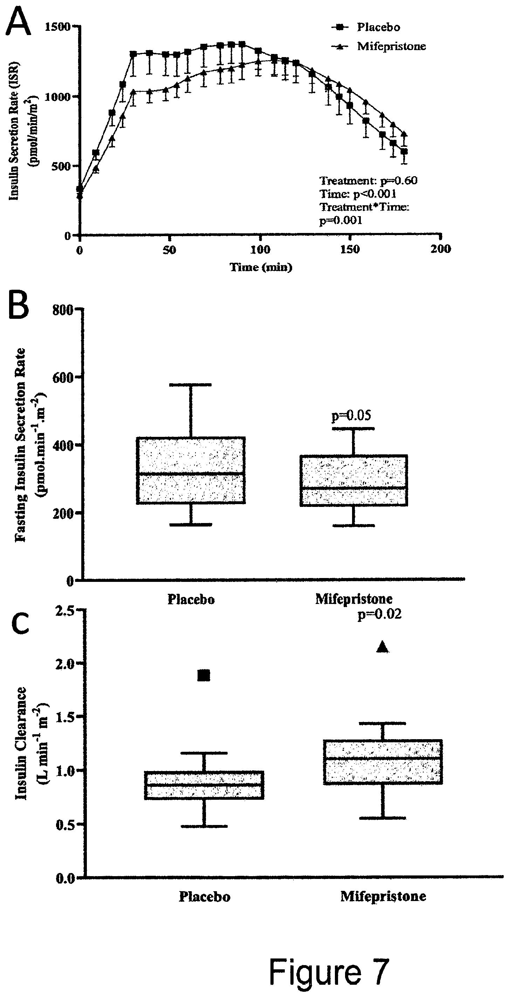

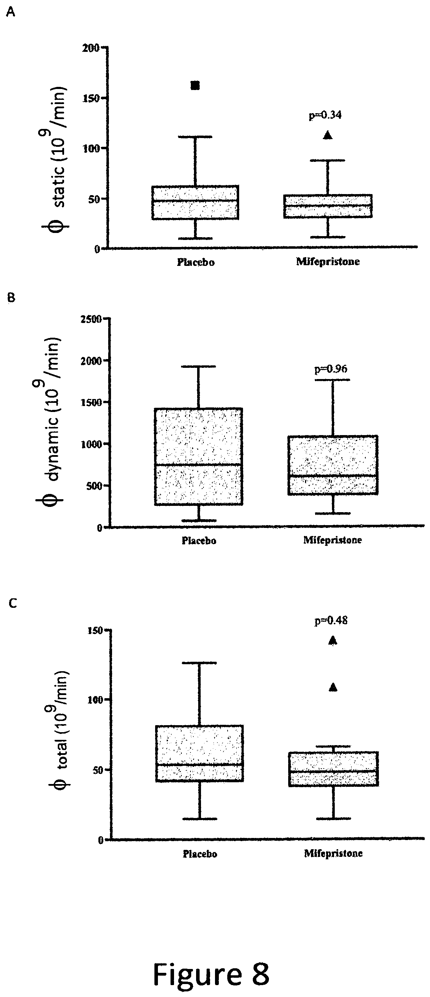

Insulin secretory rates (ISR) were derived by mathematical modeling of C-peptide and glucose concentrations during an OGTT. ß-cell sensitivity parameters derived from the modeling include basal (Ø_{B}), first-phase (Ø₁), second-phase (Ø₂) and total (Ø_{TOT}). Fasting ISR was significantly lower following mifepristone (see Figure 7B), but the ISR during OGTT was not different. See Figure 7A. The static, dynamic and total ß-cell responsivity indices derived from OGTT were similar in the mifepristone and placebo groups. See Figure 8. Insulin clearance during OGTT was lower following mifepristone when compared with placebo. See Figure 7B.

### No Serious Adverse Effects Associated With Mifepristone and Placebo Administration:

No serious adverse events were reported by subjects or detected independently during the experiments in this Example. The safety laboratory results remained within normal limits except for two subjects with increased alanine transferase after mifepristone and one patient before and after, and another after placebo administration. Abnormal values were less than one-fold the upper limit of normal 40 U/L, ranging from 41 to 47 U/L.

Example 2 demonstrates the effects of mifepristone on insulin sensitivity in overweight and obese individuals who had no secondary reason for abnormal glucose control, such as an adrenal adenoma or diabetogenic medication. This point reinforces the fact that the use of one or more GR antagonists as described herein to improve insulin sensitivity is effected under non-rescue conditions, i.e., secondary reasons for abnormal glucose control other than being overweight or obese.

Specifically, the present inventors have demonstrated for the first time in such a population (overweight and/or obese) that short term GR antagonism had variable effects on insulin sensitivity, with a significant improvement at the adipose tissue, a tendency to such improvement at the liver, and no significant difference in overall peripheral insulin sensitivity. These salutary changes were accompanied by significant decreases in fasting glucose and insulin levels and the insulin area under the curve (AUC) values during an OGTT. The improvements in glycemic control can be attributed to mifepristone, as weight did not change. We surmise that the increased adipocyte sensitivity, with decreased lipolysis (as shown by decreased FFA excursions), led to partial improvement in hepatic sensitivity, and that with longer exposure to one or more GR antagonists, whole body insulin sensitivity would improve. However, the present invention explicitly contemplates improving insulin sensitivity in individuals who are neither overweight nor obese. It has been estimated that about 12% of people in the U.S. with diabetes are normal weight. Such people may have as much fat as someone who is overweight, but the fat of such normal weight individuals is visceral fat.

The study in Example 2 demonstrates many important features of the present invention. Further, the cross-over experimental design reduced the potential for inter-person variability and increased the ability to detect treatment differences in the modest sample size (n = 16). Also, the use of a wash-out period of 6-8 weeks provided a minimization of carryover effects of treatment. As noted, efforts were made to maintain study subjects on isocaloric diets with a stable exercise routine with no new medications being allowed to minimize influences on period effects.

Furthermore, the present inventors have used a low total daily dose of mifepristone to avoid drug-induced adrenal insufficiency or other side effects. Additionally, mifepristone was administered four (4) times daily based upon the hypothesis that such a regimen would take advantage of a differentially higher exposure of the pituitary gland compared to the omentum and liver. The present inventors postulated that the first pass effect of an oral dose of mifepristone would provide a larger mifepristone "dose" to the pre-hepatic and hepatic tissues, and reduce 11-ßHSD1 activity, before hepatic metabolism resulted in a smaller circulating level that would be recognized at the pituitary gland and stimulate ACTH. This objective was achieved as evidenced by the fact that UFC values were normal or within the expected stress range. Presumably, that stress range of UFC was sufficient to be blocked by the GR antagonist, so that peripheral cortisol effects (apart from pre-hepatic effects) were normal.

### Pharmaceutical Compositions

Pharmaceutical compositions may be formulated as single, binary or ternary compositions depending on whether ER agonists and/or progestin agonists are added to the one or more GR antagonists as disclosed herein. Further, any of these compositions may be formulated using any of the excipients disclosed in the U.S. patents described herein.
These excipients are all necessarily pharmaceutically-acceptable.

For example, any of the excipient formulations in U.S. patent 10,238,659 may be used with one or more GR antagonists as disclosed herein in accordance with the present invention. However, certain excipient formulations of this patent may be more preferable than others based on solubility characteristics of mifepristone, employed in the formulation.
Determining the optimal excipient formulation is within the ordinary skill of the artisan. See U.S. 10,238,659, as noted above.
While any method of administration many be employed, oral administration is one of the preferred methods of administration. Hence the one of more GR antagonists described herein may be administered in the
form of tablets or capsules with the one or more GR antagonists being included in amounts of from about 25 to 75 mg per single dose. Such doses are typically administered several times daily as described herein to improve insulin sensitivity and/or lose weight, for example.

All GR antagonists described herein are all known, and can either be purchased commercially or may be synthesized by known synthetic methodologies.

## Claims

1. A glucocorticoid receptor antagonist for use in a method of ameliorating a disorder of insulin sensitivity in a patient,
wherein said disorder is selected from the group consisting of metabolic syndrome, diabetes, prediabetes, lipodystrophy, hypertriglyceridemia, non-alcoholic fatty liver disease, and growth hormone excess states, and wherein said method is not conducted i) for the purpose of alleviating the side effects of a medication, or ii) to provide rescue from the effects of cessation of exercise and/or caloric restriction, or iii) to treat cancer, or iv) wherein the patient has excessive or dysregulated cortisol secretion;
wherein the glucocorticoid receptor antagonist is mifepristone; and wherein the patient is administered with the glucocorticoid receptor antagonist at a dose of 30-70mg, 3 to 5 times a day.

2. The glucocorticoid receptor antagonist for use according to claim 1, wherein 50 mg mifepristone is administered every 6 hours with 200 mg being the total daily dose.

3. The glucocorticoid receptor antagonist for use according to claim 1, wherein said glucocorticoid receptor antagonist is to be administered by a route that is oral, transdermal, topical, intra-vaginal, rectal, by suppository, or by depot.

## Patentansprüche

1. Ein Glukokortikoidrezeptorantagonist zur Verwendung in einem Verfahren zur Verbesserung einer Insulinsensitivitätsstörung in einem Patienten,
wobei die Störung aus der Gruppe bestehend aus Metabolischem Syndrom, Diabetes, Prädiabetes, Lipodystrophie, Hypertriglyceridämie, Nicht-alkoholischer Fettleber, und Wachstumshormonüberschusszuständen ausgewählt ist, und wobei das Verfahren nicht durchgeführt wird i) zum Zwecke der Linderung von Nebenwirkungen einer Medikation, oder ii) um vor den Auswirkungen der Einstellung eines Trainings und /oder einer Kalorienbeschränkung zu bewahren oder iii) um Krebs zu behandeln, oder iv) wobei der Patient eine exzessive oder deregulierte Cortisolsekretion aufweist;
wobei der Glukokortikoidrezeptorantagonist Mifepriston ist; und wobei dem Patienten eine Dosis des Glukokortikoidrezeptorantagonisten von 30-70 mg, 3 bis 5 mal am Tag, verabreicht wird.

2. Der Glukokortikoidrezeptorantagonist zur Verwendung nach Anspruch 1, wobei 50 mg Mifepriston alle 6 Stunden mit einer Tagesgesamtdosis von 200 mg verabreicht wird.

3. Der Glukokortikoidrezeptorantagonist zur Verwendung nach Anspruch 1, wobei der Glukokortikoidrezeptorantagonist auf oralem, transdermalem, topischem, intravaginalem, rektalem Wege oder als Zäpfchen oder Depot verabreicht wird.

## Revendications

1. Antagoniste du récepteur des glucocorticoïdes pour une utilisation dans une méthode consistant à soulager un trouble de la sensibilité à l'insuline chez un patient,
dans lequel ledit trouble est choisi dans le groupe consistant en un syndrome métabolique, le diabète, le pré-diabète, la lipodystrophie, l'hypertriglycéridémie, la stéatose hépatique non alcoolique et des états d'excès d'hormone de croissance, et dans lequel ladite méthode n'est pas réalisée i) aux fins d'éviter les effets secondaires d'un traitement médicamenteux, ou (ii) d'apporter un secours vis-à-vis des effets de l'arrêt de l'activité sportive et/ou de restrictions caloriques, ou (iii) pour traiter le cancer, ou iv) dans un cas où le patient présente une sécrétion de cortisol excessive ou dérégulée ; dans lequel l'antagoniste du récepteur des glucocorticoïdes est la mifépristone ; et dans lequel le patient reçoit une administration de l'antagoniste du récepteur des glucocorticoïdes à une dose de 30 à 70 mg, 3 à 5 fois par jour.

2. Antagoniste du récepteur des glucocorticoïdes pour une utilisation selon la revendication 1, dans lequel 50 mg de mifépristone sont administrés toutes les 6 heures, 200 mg étant la dose quotidienne totale.

3. Antagoniste du récepteur des glucocorticoïdes pour une utilisation selon la revendication 1, où ledit antagoniste du récepteur des glucocorticoïdes doit être administré par une voie qui est la voie orale, transdermique, topique, intravaginale, rectale, par suppositoire, ou par retard.
